# EUROPÄISCHE PATENTSCHRIFT

(11) **EP 3 463 052 B1**
(45) Veröffentlichungstag und Bekanntmachung des Hinweises auf die Patenterteilung: **16.09.2026**
(21) Anmeldenummer: 17732757.4
(22) Anmeldetag: 24.05.2017
(51) Int. Cl.: A61B 5/00, A61B 5/318, A61B 5/369

(54) **TEXTILPRODUKT MIT HAUTKONTAKTELEMENT UND/ODER AUSSENSEITIGER KONTAKTIERUNG DES HAUTKONTAKTELEMENTS, UND VERFAHREN ZU SEINER HERSTELLUNG**
TEXTILE PRODUCT WITH SKIN-CONTACT ELEMENT AND/OR ESTABLISHING EXTERNAL CONTACT WITH THE SKIN-CONTACT ELEMENT, AND METHOD FOR PRODUCING THE SAME
PRODUIT TEXTILE PRÉSENTANT UN ÉLÉMENT DE CONTACT AVEC LA PEAU ET/OU UN CONTACT EXTERNE DE L'ÉLÉMENT DE CONTACT AVEC LA PEAU ET PROCÉDÉ POUR SA FABRICATION

(30) Priorität: 25.05.2016 DE 102016109719; 23.09.2016 DE 102016118001
(43) Veröffentlichungstag der Anmeldung: 10.04.2019
(73) Patentinhaber: M3S GmbH, 82211 Herrsching (DE)
(72) Erfinder: STRECKER, Markus, 82211 Herrsching (DE)
(74) Vertreter: Strehl & Partner mbB
(86) Internationale Anmeldenummer: PCT/EP2017/062570
(87) Internationale Veröffentlichungsnummer: WO 2017/202928

(56) Entgegenhaltungen:
- EP-A1- 2 679 107
- WO-A1-2008/022482
- WO-A1-2009/043196
- US-A1- 2015 040 282
- US-A1- 2015 250 420

## Beschreibung

### Gebiet der Erfindung

Die vorliegende Erfindung betrifft ein Textilprodukt mit eingearbeitetem Hautkontaktelement, insbesondere mit einem eingearbeiteten Hautkontaktelement und/oder einer eingearbeiteten Elektrode, der/die beispielsweise als Sensor zur Messung von Herzfrequenzen, Atemfrequenzen, Hauttemperatur und dergleichen dienen kann. Sie betrifft insbesondere auch die Verbindungstechnik zwischen dem Hautkontaktelement und einer elektronischen Mess- und Auswerte-Einheit. Insbesondere handelt es sich dabei um ein Hemd oder Shirt. Die Erfindung betrifft auch ein Verfahren zur Herstellung eines Textilprodukts.

### Stand der Technik

In Sport, Freizeit und Healthcare besteht der Wunsch vieler Menschen, ihre biometrischen Daten aufzunehmen und diese zur Verbesserung ihrer sportlichen Leistungsfähigkeit oder zur Überwachung ihres Gesundheitszustandes einzusetzen. Einer der wichtigen Parameter ist die Messung der Herzfrequenz sowie der Herzfrequenzvariabilität. Diese werden heute überwiegend mit einem Brustgurt erfasst. Auch Geräte, die diese Parameter über optische Sensoren messen, sind heute üblich. Diese Geräte sind jedoch zumeist nicht präzise genug.

Der Brustgurt wird von vielen Menschen als unbequem empfunden. Bei Marathonläufern wird er aufgrund der Reibung und Irritation der Haut häufig gerne vermieden.

Es gibt auf dem Markt eine Reihe von T-Shirts mit eingearbeiteten Elektroden. Diese werden zumeist an ein konventionelles Elektronikmodul angebunden. Dieses ist typischerweise zwischen oder neben den Elektroden angebracht und über Druckknöpfe mit dem Shirt verbunden. Diese Anordnung ist vor allem für Ballsportler nicht denkbar, zum Beispiel für Fußballspieler.

Die bestehenden Shirts haben heute meist Elektroden aus leitfähigem Polymermaterial, das nicht dehnbar ist und somit Bewegungen des Shirts gegenüber dem Körper forciert. Das Shirt macht nicht alle Bewegungen harmonisch mit. Es gibt auch Ausführungen von Shirts mit einer Elektrode aus silberbeschichtetem Textilmaterial. Dieses Material hat aber wesentliche Einschränkungen, was die Haltbarkeit anbelangt. So ist es beim Waschen anfällig gegenüber Chlorbestandteilen in der Waschlauge. Schweiß in Verbindung mit schwachen elektrischen Signalen genügt, um die Silberbeschichtung schnell in Ihrer Funktion zu beeinträchtigen. Auch in der Funktion haben die silberbeschichteten Stoffe wesentliche Einschränkungen, denn sie benötigen eine Mindestmenge an Feuchtigkeit, um einen guten Kontakt zur Haut herzustellen, und sind trocken nicht funktionsfähig. Zudem sind diese meist sehr teuer und komplex in der

Fertigung. Bekannte Textilien zur Messung von verschiedenen Parametern sind aus US 2015/040282 A1, US 2015/250420 A1, WO 2008/022482 A1, WO 2009/043196 A1 und EP 2 679 107 A1 bekannt.

Elektroden werden Personen beispielsweise angelegt, um ein EKG (Elektrokardiogramm) zu messen. Ein EKG ist ein Oszillogramm, mit dem Muskelaktivitäten, auch die der Herzmuskel, elektrisch dargestellt werden. Das grundlegende Prinzip der EKG-Messung basiert auf der Messung von Spannungsgefällen zwischen menschlichen Extremitäten. Beim EKG misst man die Potenzialverteilung an der Oberfläche des menschlichen Körpers, die sich durch zeitliche Überlagerung aller Nervensignale im Herzen ergibt.

Zur EKG- und Pulsmessung müssen in Medizin und Sport elektrische Signale von der Hautoberfläche abgelesen werden. Langzeit-EKGs sind besonders für die Diagnostik von Herzrhythmusstörungen wichtig, sie können mittels herkömmlicher Klebe-Elektroden jedoch nur bis zu ca. 24 Stunden durchgeführt werden. Im Sport werden Brustgurte zur Pulsmessung eingesetzt, die nicht nur störend wirken, sondern auch meist nur bei Verwendung eines Leitgels zuverlässig arbeiten.

Der Nachteil der Prozedur beim EKG ist, dass zunächst ein Gel auf dem Brustkorb des Patienten verteilt werden muss, um den ohmschen Kontakt der Elektroden mit der Haut herzustellen. Dann muss der Patient ruhig liegen, damit keine Elektrode abfällt.

Das zu messende Signal liegt im Bereich einiger Millivolt. Anschließend verstärken, digitalisieren und filtern analoge Frontends (AFEs) und DSPs die analogen, bioelektrischen Sensorsignale. Hat die Signalverarbeitung diese Daten entsprechend aufbereitet, können sie beispielsweise per Bluetooth übertragen werden. Je nach Einsatzbereich in den Bereichen Sport und Fitness oder in der Echtzeit-Telemedizin sind unterschiedlichste drahtgebundene oder drahtlose Übertragungswege von bioelektrischen Signalen an ein Armband oder eine Uhr, oder sogar direkt über eine größere Distanz möglich, die dann am PC zur Selbstkontrolle der eigenen körperlichen Fitness oder zur Gesundheitskontrolle von einem Arzt ausgewertet werden können.

Mit Hilfe textiler oder Kunststoff- (z.B. PA-, PU- oder Silikon-) basierter Sensoren könnten EKG- und Puls-Signale zeitlich unbegrenzt und ohne Hautirritationen hervorzurufen gemessen werden. Solche Elektroden bestehen aus textilen Leitern, beispielsweise Umwindegarnen, Fasergarnen mit Edelstahlanteil oder leitfähig beschichteten Garnen.

Diese Garne besitzen einen textilen Charakter, weil man die geringen metallischen Anteile nicht spürt, und sie lassen sich wie jedes andere Garn verweben, aufsticken oder stricken.

Diese textilen Elektroden können in ein Sensor-Textilprodukt integriert werden, um EKG oder Puls zu messen. Hierdurch werden sie gleichzeitig korrekt positioniert; ein fehlerhaftes Anlegen ist schwer möglich.

Ein weiteres Produkt des Standes der Technik ist beispielsweise ein Reizstrom-Gerät, das an das Handgelenk und dergleichen angelegt werden kann. Für bestimmte Körperstellen wie die Handgelenke braucht man Spezial-Elektroden, die sanft anliegen und alles umschließen. Dieses Ziel wird erreicht durch die Verwendung von elastischen Manschetten, in die Silbergarn eingearbeitet wurde. Dieses überträgt den Reizstrom gleichmäßig und hat eine lange Haltbarkeit.

Der Nachteil diese Produkte ist jedoch, dass die Elektroden als solche nicht dehnbar sind, oder anfällig gegen Chlor oder die Haut irritieren, bzw. auch oft sehr dick sind und nicht dehnbar sind. Weiterhin müssen die elektrischen Verbindungsstellen meist mit nicht dehnbaren Materialien vergossen werden, bzw. relativ dick eingekapselt werden. Dies führt zu einer deutlichen Einschränkung der Verwendbarkeit und des Tragekomforts dieser Produkte.

### Durch die Erfindung zu lösende Aufgaben

Es ist Aufgabe der vorliegenden Erfindung, ein Textilprodukt bereitzustellen, das eine oder mehrere Hautkontaktelemente wie beispielsweise Elektroden und/oder (flache) Sensoren oder Aktuatoren zur Messung von Körperaktivitäten, Temperatur oder zur Übertragung von elektrischem Strom oder anderen Reizen wie beispielsweise Vibrationen von der Haut oder auf die Haut eines Trägers aufweist und hinsichtlich des Tragekomforts Vorteile bietet.

### Zusammenfassende Darstellung der Erfindung

Die Aufgabe wird durch Bereitstellen des erfindungsgemäßen Textilprodukts gelöst.

Der Gegenstand der vorliegenden Erfindung ist durch die angehängten Ansprüche definiert.

### Vorteile der Erfindung

Die Erfindung hat in ihren Ausgestaltungen mit dehnbarer Elektrode den wesentlichen Vorteil, dass durch die Dehnbarkeit der dehnbaren Elektrode der Tragekomfort des Textilprodukts durch die Anwesenheit der Elektrode nicht oder nur fast unmerklich oder unwesentlich eingeschränkt ist.

Ein weiterer, wesentlicher Vorteil liegt darin, dass die Elektrode so gestaltet bzw. der Sensor so angeordnet ist, dass sie/er an ihrer/seiner Außenseite ankontaktiert werden kann. Da diese Außenseite in der Regel nicht im Inneren des Textilprodukts liegt, kann die Führung der elektrischen Leitungen auf der Außenseite des Textilmaterials erfolgen, was den Tragekomfort extrem erhöht und kein Reiben der elektrischen Leitung auf der Haut verursacht.

Außerdem hat das erfindungsgemäße Textilprodukt den Vorteil, dass die Fertigungstechnik weitgehend mit verschiedenen Standardfertigungsmethoden in der Textilfertigung kompatibel ist und praktisch zusätzlich zu diesen angewandt werden kann. Dadurch können unter anderem die Herstellungskosten reduziert werden.

### Eingehende Beschreibung der Erfindung

### Leitfähiger Kunststoff

Silikone sind polymere Verbindungen, die eine Zwischenstellung zwischen anorganischen und organischen Verbindungen einnehmen. Während bei den vollständig organischen Polymeren hauptsächlich Kohlenstoffatome die Molekülkette bilden, ist es bei den Silikonen das Silicium. Das typische Merkmal der Silikone ist die Siloxan-Bindung Si-O-Si, weswegen auch die Bezeichnung Polysiloxane häufig verwendet wird. Ihren organischen Charakter erhalten die Silikone durch die an die Siliciumatome gebundenen Kohlenwasserstoffgruppen. Das sind meistens Methylgruppen, seltener Ethyl-, Propyl-, Phenyl- und andere Kohlenwasserstoffreste. Die Silikone können je nach Kettenlänge, Verzweigungsgrad und Art der am Silicium gebundenen Kohlenwasserstoffgruppen flüssig bis zähflüssig oder fest sein. Die meisten Silikone sind Wasser abstoßend, elektrische Isolatoren und beständig gegen Säuren. Sie sind nicht gesundheitsschädlich.

Silikonkautschuke sind in den gummielastischen Zustand überführbare Massen, welche Poly(organo)siloxane enthalten, die für Vernetzungsreaktionen zugängliche Gruppen aufweisen. Als solche kommen vorwiegend Wasserstoffatome, Hydroxygruppen und Vinylgruppen in Frage, die sich an den Kettenenden befinden, aber auch in die Kette eingebaut sein können. Silikonkautschuke enthalten in der Regel verstärkende Stoffe und Füllstoffe, deren Art und Menge das mechanische und chemische Verhalten der durch die Vernetzung entstehenden Silikonelastomere deutlich beeinflussen. Silikonkautschuke können mit geeigneten Pigmenten gefärbt werden.

Man unterscheidet nach der notwendigen Vernetzungstemperatur zwischen kalt- (RTV) und heißvernetzenden (HTV) Silikonkautschuken (RTV = raumtemperatur vernetzend, HTV = hochtemperatur vernetzend). HTV-Silikon-Kautschuke sind plastisch verformbare Materialien. Sie enthalten sehr oft organische Peroxide für die Vernetzung. Die daraus durch die Vernetzung bei hoher Temperatur hergestellten Elastomere sind wärmebeständige, zwischen -40 und 250 °C elastische Produkte, die beispielsweise als hochwertige Dichtungs-, Dämpfungs-, Elektroisolierbauteile, Kabelummantelungen und dergleichen verwendet werden.

Die in der vorliegenden Erfindung einsetzbaren elektrisch leitfähigen elastischen Polymere sind nicht besonders eingeschränkt, solange sie die gewünschten elastischen Eigenschaften aufweisen. Sie sind jedoch vorzugsweise auf Silikonbasis hergestellt.

Beispielsweise bestehen sie aus Silikon und/oder Fluorsilikon sowie leitfähigen Partikeln.

Das leitfähige Material umfasst eine Bandbreite von Kohlenstoffpartikel wie Ruß bis Metallpartikeln wie z.B. Silber. Die in das Polymer, z. B. Silikon, eingeführten leitfähigen Partikel sind beispielsweise Metalle, wie Eisen, Silber, Kupfer oder Gold, oder Kohlenstoff enthaltende Partikel, z.B. in der Form von Graphit, Graphen, Kohlenstofffasern oder Nanoröhrchen. Auch leitfähige Polymere wie Polypyrrol oder Polythiophen sind möglich.

Der Ausdruck "Partikel" bedeutet in der vorliegenden Erfindung Teilchen mit einem Durchmesser von 10 nm bis 10 µm, vorzugsweise 100 nm bis 5 µm.

Vorzugsweise werden als Elektrodenmaterial und als leitfähiges Material Silikon-Elastomere (Silikongummi) eingesetzt, die bevorzugt durch den Einbau von Kohlenstoff elektrisch leitfähig gemacht worden sind.

Beispielsweise kann ein Gemisch aus Kohlenstoff-Nanoröhrchen und Silikon-Polymeren eingesetzt werden, das hohe elektrische Leitfähigkeit zeigt. Diese Kunststoffe sind sowohl elektrisch leitfähig und flexibel als auch dehnbar. Ein Anteil von beispielsweise bis zu 20 Gewichtsprozent an einwandigen Nanoröhrchen verringert nicht die mechanische Flexibilität und Weichheit des Polymers. Das Gummi ist ein dehnbares Polymer auf der Basis von Dimethyl-Siloxan. Mit diesem wird eine zweite dünne Kunststoffschicht überzogen, in der vorher die elektrisch leitfähigen Nanoröhrchen verteilt wurden. Um die störenden Verklumpungen in einem Gemisch aus den winzigen Kohlenstoffpartikeln in einem Polymer zu vermeiden, werden die Nanoröhrchen vorher in einer ionischen Flüssigkeit (1-Butyl-3-Methylimidazoliumbisimid) verteilt. Diese Substanz verhindert effektiv, dass sich die Röhrchen aneinanderlagern. Das so entstandene schwarze Nanogel wird dann dann mit einem weiteren flüssigen Polymer (Vinylidenfluoridhexafluoropropylen) vermengt und die erhaltene Mischung wird als dünner Film auf einen Glasträger aufgesprüht. Dieses Kompositmaterial wird dann mit dem dehnbaren Silikongummi zum gewünschten Endprodukt verbunden. Dieser Werkstoff zeigt im ungedehnten Zustand eine gute Leitfähigkeit und lässt sich viele Male auseinander ziehen, ohne dass die Leitfähigkeit abnimmt. So können auf relativ einfache Weise sehr strapazierfähige Materialien für die erfindungsgemäßen Textilprodukte hergestellt werden.

Alternativ kann auch Graphen eingearbeitet werden, um die erforderliche Leitfähigkeit zu bewirken.

Der in der vorliegenden Erfindung verwendete Ausdruck "dehnbar" wird identisch mit Begriff "elastisch" verwendet. Elastizität ist gemäß allgemeiner Definition die Eigenschaft eines Körpers oder Werkstoffs, unter Krafteinwirkung seine Form zu verändern und bei Wegfall der einwirkenden Kraft in die Ursprungsform zurückzukehren. In der vorliegenden Erfindung bedeutet dies beispielsweise, dass ein Textilmaterial oder eine Elektrode durch eine Zugbeanspruchung in mindestens eine Richtung gedehnt werden kann und nach Beenden der Zugbelastung in seine ursprüngliche Form zurückkehrt. In der vorliegenden Erfindung sind die elastischen Eigenschaften, wie die Dehnbarkeit und die Elastizitätsgrenze, des Textilmaterials und der Elektrode vorzugsweise identisch.

Der hier verwendete Ausdruck "dehnbares Elektrodenmaterial" bezeichnet eine Zusammensetzung, welche die genannten leitfähigen Partikel und den genannten dehnbaren Kunststoff enthält. Der dehnbare Kunststoff wird so ausgewählt, dass er nach einem gegebenenfalls durchgeführten Vernetzungsvorgang dehnbar und für die vorliegende Erfindung geeignet ist. Die Dehnbarkeit liegt vorzugsweise bei mindestens 10 %, stärker bevorzugt bei mindestens 30 % und noch stärker bevorzugt bei mindestens 50% in mindestens einer, vorzugsweise beiden Flächenrichtungen.

Das in der vorliegenden Erfindung eingesetzte Elektrodenmaterial ist nicht besonders eingeschränkt, solange es die Aufgabe als Elektrode erfüllen kann. Vorzugsweise enthält das Elektrodenmaterial einen dehnbaren Kunststoff. Ein besonders bevorzugtes Material ist Silikon, das elektrisch leitende Partikel enthält. Besonders bevorzugt besteht die Elektrode aus einem mit Kohlenstoffpartikeln beladenen Silikon und/oder besitzt eine Dicke von 100 bis 500 µm.

Das in der vorliegenden Erfindung eingesetzte härtbare Kunststoffmaterial, mit dem das Textilmaterial versetzt wird, kann jedes Kunststoffmaterial sein, das beispielsweise durch Erwärmen härtbar ist. Vorzugsweise ist dieses Kunststoffmaterial ein Silikon.

Das in der vorliegenden Erfindung eingesetzte Klebemittel ist nicht besonders eingeschränkt, solange die gewünschten Materialien verklebt und damit fest verbunden werden können. Das Klebemittel ist vorzugsweise ein härtbares Kunststoffmaterial, das beispielsweise durch Erwärmen härtbar ist. Vorzugsweise ist dieses Kunststoffmaterial ein Silikon. Stattdessen ist es auch möglich, einen Schmelzkleber einzusetzen, der auf das Textilmaterial aufgebracht wird und bei Umgebungstemperatur nicht klebt. In einer Heißpresse oder dergleichen kann er in den klebenden Zustand überführt werden. Vorteilhaft an einem derartigen Kleber ist, dass Überschüsse des Klebers, die von den zu verklebenden Materialien nicht beidseitig abgedeckt werden, keine Klebaktivität mehr zeigt, sobald wieder Umgebungstemperatur und -druck herrschen. Materialien für Schmelzkleber sind dem Fachmann bekannt. Beispielsweise kann PU (Polyurethan) eingesetzt werden.

In einer Ausführungsform besteht die Elektrode aus einem Silikon-Material. Dieses ist mit Carbon-Partikeln geladen und somit elektrisch leitend. Die Dicke der Elektrode ist im Bereich von 100 bis 500 µm, vorzugsweise 250 bis 350 µm.

### Einbringen der Elektroden bzw. sonstige Sensoren

Das Elektrodenmaterial wird in einem Rakelprozess flächig auf eine Unterlage aufgebracht, und es wird ein Bogen, also eine dünne flächige Struktur, hergestellt. Dieser Bogen wird dann vereinzelt, so dass Plättchen mit einer Länge und Breite im Zentimeter- oder Millimeterbereich und einer Dicke im genannten Bereich, also beispielsweise etwa 300 µm, entstehen.

Die Elektroden und Sensoren können auf mehrere Arten eingebracht werden.

In der ersten Ausführungsform wird in den Stoff eines Textilmaterials ein Loch geschnitten. Auf den Stoff des Textilmaterials wird in der Größe der Elektrode ein klebfähiges, noch nicht vernetztes, nichtleitendes Silikon aufgetragen. Das Loch wird mit einem Stück Stoff als Träger hinterfüttert. Dieser hinterfütterte Stoff wird vor dem Trocknen des Silikons, meist nach, aber auch manchmal bereits vor dem Aufkleben der Elektrode oder des sonstigen Sensors entfernt. Die Elektrode bzw. der Sensor wird in das noch nicht vernetzte Silikon gelegt und so verklebt. Handelt es sich um einen Sensor mit einseitiger Sensorfläche, so sollte diese nach oben weisen, damit sie sich im fertigen Zustand auf der Innenseite des Textilprodukts befindet. Im Bereich des Loches wird mittels eines leitfähigen Silikons eine Verbindung zur metallischen Seele eines dehnbaren Leitungsbändchens hergestellt. Diese Leitungsbändchen kann ein Stoffbändchen sein. Danach wird diese Kontaktstelle mit nichtleitendem Silikon abgedeckt und mit einem Stoffpatch überdeckt. Anschließend werden die noch nicht vernetzten Silikonmaterialien durch Wärme oder Licht vernetzt und damit verfestigt.

In der vorliegenden Anmeldung werden die Ausdrücke "Vernetzen" und "Härten" von Polymeren synonym verwendet.

In der zweiten Ausführungsform wird lediglich ein Schlitz in das Textilmaterial geschnitten. Die Elektrode / der Sensor wird mittels eines leitfähigen Silikons mit der metallischen Seele eines dehnbaren Leitungsbändchens verklebt, und ein Textilpatch wird über der Klebestelle aufgebracht. Die Elektrode oder ein Teil der Elektrode wird nun durch den Schlitz geschoben und dann mit Hilfe eines nichtleitenden Silikons auf der Innenseite verklebt.

Eine dritte Ausführungsform betrifft nur die Ausgestaltung der Erfindung, in der das Textilprodukt mit einer dehnbaren Elektrode ausgestattet ist. Zur Herstellung wird in dieser Ausführungsform ein Bereich des Stoffes vorzugsweise etwas gespannt und dann in allen Fällen mit leitfähigem Silikon abgedeckt, anstatt ein Loch oder einen Schlitz wie in den ersten beiden Ausführungsformen vorzusehen. Beispielsweise wird danach im Bereich unter dem Silikon auf der Rückseite des Stoffes ein Vakuum angebracht, so dass das leitfähige Silikon durch die Stoffstruktur nach innen gesaugt wird. Es entsteht nun ein Bereich des Stoffes, der mit leitfähigem Silikon durchtränkt ist. Dadurch wird eine Kontaktierung vom Innen- zum Außenbereich des Stoffes hergestellt. Auf dem Außenbereich wird vor dem Aushärten des leitfähigen Silikons ein Kontakt zu einer Zuleitung hergestellt. Beispielsweise dienen hierzu leitfähige dehnbare Bändchen, die weiter unten näher beschrieben werden. Nach dem Aushärten des Silikons kann in einigen Fällen der Erfindung auf der Innenseite ein nicht leitendes Silikon über einen Fensterprozess aufgebracht werden. Das Fenster spart den Bereich des leitfähigen Silikons aus. Dann kann eine leitfähige Elektrode eingeklebt werden.

Der oben beschriebene Prozess kann wahlweise auch mit einem anderen leitfähigen Polymermaterial durchgeführt werden.

Als Textilmaterial dient in der vorliegenden Erfindung bevorzugt ein einlagiges Textil, welches weiter bevorzugt gewirkt sein kann.

In einer Ausführungsform wird um das leitfähige Silikon (oder anderes leitfähiges Polymermaterial), das als "Durchkontaktierung" dient, noch ein Ring mit nichtleitfähigem Silikon (oder anderes leitfähiges Polymermaterial) eingebracht um die Verbindungsstelle nach außen zu isolieren.

Um eine sichere Kontaktierung der Elektrode mit der Kontaktstruktur sicherzustellen, wird als Option noch eine kleine Menge leitendes Silikon über dem leitfähigen Material aufgebracht. Auf der Außenseite wird nun nicht leitfähiges Silikon aufgebracht und dieses mit einem Stoff abgedeckt.

In einigen Fällen der Erfindung kann in dieser Ausführungsform auf das Vorsehen der einzuklebenden leitfähigen Elektrode verzichtet werden, da das den Stoff durchdringende Silikon genügend Leitfähigkeit besitzt, um die elektrischen Signale von innen nach außen zu führen, und damit bereits als solches als Elektrode fungiert.

In der dritten Ausführungsform wird ein Textilprodukt erhalten, worin die Elektrode und/oder das leitfähige Material integraler Bestandteil des Textilprodukts ist, indem die Elektrode und/oder das leitfähige Material das Gewebe des Textilprodukts mikroskopisch durchsetzt.

Das Eindringen des dehnbaren Elektrodenmaterials, welches die leitfähigen Partikel und den dehnbaren Kunststoff enthält, in das dehnbare Textilmaterial kann erleichtert werden, indem eine oder mehrere der folgenden Maßnahmen durchgeführt wird/werden:
(a) Das Elektrodenmaterial kann verdünnt werden, indem es mit einem Lösungsmittel bzw. mit zusätzlichem Lösungsmittel versetzt wird, so dass die Viskosität des Materials verringert wird und es dadurch leichter in die Poren des Textilmaterials eindringen kann. Das Lösungsmittel kann so ausgewählt werden, dass es je nach Bedarf und je nach Beschaffenheit des Textilmaterials die Hydrophobizität des Elektrodenmaterials beeinflusst.
(b) Das Textilmaterial kann gedehnt werden, so dass seine Poren vergrößert werden und dadurch das Elektrodenmaterial leichter eindringen kann. Diese Maßnahme ist dann besonders zu empfehlen, wenn die in dem Elektrodenmaterial enthaltenen leitfähigen Partikel relativ groß sind. Durch das Dehnen kann verhindert werden, dass sich diese Partikel und/oder der dehnbare Kunststoff ungleichmäßig in dem Textilmaterial verteilen. Es ist daher bevorzugt, dass die Partikel möglichst klein sind und besonders bevorzugt einen Durchmesser von 2 µm, stärker bevorzugt von 100 nm nicht überschreiten.
(c) Das Eindringen des Elektrodenmaterials kann durch eine Maßnahme erleichtert werden, bei der eine äußere Kraft auf die Materialien einwirkt. Beispielsweise kann ein Unterdruck auf einer Seite des Textilmaterials angelegt werden, so dass das Eindringen des auf der anderen Seite des Textilmaterials befindlichen Elektrodenmaterials bewirkt wird. Ein anderes Beispiel ist das Aufdrucken des Elektrodenmaterials.
(d) Das Elektrodenmaterial kann zur Verringerung seiner Viskosität erwärmt werden.

Ein wesentliches Merkmal der Elektrodenanordnung der vorliegenden Erfindung ist, dass die Elektrode von der Innenseite des Textilmaterials zu dessen Außenseite geführt ist. Außerdem wird die Verbindungsleitung an der Außenseite des Textilprodukts geführt, wodurch der Tragekomfort für den Nutzer erhöht wird. Besonders bevorzugt werden alle Teile, die auftragen, also eine Erhebung auf dem Textilprodukt darstellen und damit stören können, außen angeordnet. Ein weiteres Merkmal ist das Herstellen einer dehnbaren und flexiblen elektrischen Verbindung von innen nach außen, wodurch die Flexibilität und Dehnbarkeit des Textils erhalten bleibt.

Die in dem erfindungsgemäßen Textilprodukt einsetzbare Elektrode dient der Messung von elektrischen Strömen am Körper des Trägers des Textilprodukts, zur Stimulation des Körpers über elektrische Impulse/Ströme/Spannungen. Alternativ kann ein Sensor in das Textilprodukt eingearbeitet werden bzw. sein, der der Messung der Hauttemperatur, der Atmung oder des Drucks dient, den der Körper des Trägers des Textilprodukts an der Stelle des Sensors gegen eine Gegenfläche ausübt. Die Elektrode bzw. der Sensor ist vorzugsweise auf der Innenseite des Textilprodukts angeordnet, also auf der Seite, die dem Körper des Trägers des Textilprodukts zugewandt ist. Bevorzugt befindet sich die Elektrode innenseitig flächenbündig auf dem Textilprodukt. Die Kontaktierung dagegen erfolgt auf der vom Träger abgewandten Seite des Textilprodukts.

### Ableitungselement

Die Elektroden bzw. Sensoren sind über ein Ableitungselement mit dem Mess- und Auswertesystem verbunden. Das Ableitungselement ist dadurch gekennzeichnet, dass zwar einerseits als Leiter Metall verwendet wird, dass das Ableitungselement jedoch andererseits dehnbar ist, ohne dass die Gefahr besteht, dass das Metall beschädigt wird, sodass sich das Ableitungselement bei Dehnung des Textilprodukts ebenfalls dehnen kann, ohne dass dies Nachteile für den Transport der elektrischen Information hätte.

In einer ersten Variante handelt es sich bei dem Ableitungselement um ein Leitungsbändchen, besonders bevorzugt ein leitfähiges textiles Band.

Dieses textile Band oder Bändchen ist mit einer Litze (auch Seele genannt) ausgestattet. Dieses wird vorzugsweise sinusförmig bzw. wellenförmig eingewebt. Eine Besonderheit ist hier, dass mehrere, z.B. vier, zehn oder 16 oder z.B. auch 40 einzelne isolierte Drähtchen (metallische Leiter) enthalten sind und die Lackisolierung vorzugsweise mehrere verschiedene Farben aufweist, um unterschiedliche elektrische Informationen tragende Drähtchen voneinander unterscheiden zu können. Das Bändchen ist vorzugsweise so konzipiert, dass es die metallischen Leiter auf einer Seite so abdeckt, dass man sie nicht sieht. Auf der anderen Seite können sie sichtbar sein, können natürlich aber auch auf dieser Seite "versteckt" sein. In alternativen Ausgestaltungen der Erfindung kann anstelle der Lackisolation auch eine Ummantelung oder eine beispielsweise aus PFA-Material extrudierte Isolation verwendet werden.

Das Bändchen kann, muss aber nicht, dehnbar sein. Mit geeigneten Webtechniken kann z.B. eine Dehnfähigkeit von ca. 0 % (nicht dehnbar) bis 100% erreicht werden. In Ausführungsformen der Erfindung ist die Dehnfähigkeit bevorzugt 5 bis 70 Gew.-%.

Das Bändchen ist bezüglich seiner Breite und Dicke vorzugsweise so gestaltet, dass es in der Dimension mit einer Flatlockmaschine übernäht und somit befestigt werden kann. Die elektrischen Leiter sind in der Regel sinusförmig oder wellenartig in der Mitte mit einem Abstand zu den Rändern angeordnet, so dass sie nicht verletzt werden, wenn das Bändchen an den Rändern eingenäht wird. Das heißt, die Dimensionierung des Bändchens wird für den Einsatz einer Flatlock-Nähmaschine angepasst, insbesondere einer Nähmaschine mit zwei Nadeln. Mit einer Flatlock-Nähmaschine kann ein sogenannte Flachnaht oder Flatlock-Naht hergestellt werden. Diese ist vor allem eine Ziernaht. Sie hat den Vorteil, dass sie flach ist und damit bei enganliegender Kleidung keine Nahtzugabe stört.

Anstelle eines derartigen Bändchens kann in einer zweiten Variante ein dehnbarer Faden bzw. ein aus dehnbaren Fäden hergestelltes, beispielsweise gewebtes Bändchen vorgesehen sein, wobei der dehnbare Faden bzw. einzelne oder Gruppen der dehnbaren Fäden des Bändchens mit einem oder mehreren metallischen Fäden umwickelt sind. Als Fäden können z.B. Gummifäden dienen. Wird ein Bändchen verwendet, so gilt hinsichtlich seiner Dimension und der Fähigkeit, eingenäht zu werden, dasselbe, was für das Bändchen mit Litze oder Seele ausgeführt wurde.

Alternativ zur Befestigung der Leitungsbändchen durch Einnähen kann das Bändchen oder der Faden auch aufgeklebt, eingeklebt oder auflaminiert werden. Letzteres ist beispielsweise mit Hilfe eines Klebers möglich, der über eine Temperaturerhöhung aktiviert wird. In diesen Fällen ist die Dimension des Bändchens nicht beschränkt.

Vorzugsweise umfassen die Bändchen Stoffbändchen und die elektrischen Leiter, die auf einer Seite des Stoffbändchens aufgebracht sind. Die Bändchen werden dann so auf das Textilprodukt aufgebracht, dass die Stoffbändchen außen und die elektrischen Leiter dem Textilprodukt zugewandt liegen und die elektrischen Leiter damit nicht sichtbar sind.

In der vorliegenden Erfindung bedeutet der Ausdruck "Litze" ein aus dünnen Einzeldrähten bestehender und daher leicht zu biegender elektrischer Leiter. In elektrischen Kabeln wird überwiegend Kupfer als Leiter verwendet. Einzelne Drähte der Litze (einige, z.B. vier oder 10, bis mehrere hundert) können von einer gemeinsamen Isolierhülle umschlossen sein. Das Metall der darin enthaltenen elektrischen Leiter ("Drähtchen") besteht vorzugsweise aus Kupfer, Silber oder einer Legierung, die vorzugsweise Kupfer oder Silber als Bestandteil enthält. Auch kann ein mit Silber beschichteter Kupferdraht genutzt werden. Dadurch werden die Biegefestigkeit des Leiters und/oder seine Flexibilität erhöht. In weiteren vorteilhaften Ausgestaltungen der Erfindung werden Leiter aus Edelstahl verwendet.

Der Vorteil von Litzenleitungen und mit Drähtchen umwickelten dehnbaren Seelen ist, dass die Gefahr eines Leiterbruches durch Biegung wesentlich geringer ist als bei Massivdrahtleitern mit gleichem Querschnitt. Daher sind Litzenleitungen und Leitungen aus mit Drähtchen umwickelten dehnbaren Seelen für die Verwendung in Textilien, bei der eine häufige Bewegung oder Rüttelbeanspruchung stattfindet, besonders geeignet. Je nach erforderlicher Flexibilität und Beanspruchungsgrad verwendet man fein- oder feinstdrähtige Litzenleitungen.

In der vorliegenden Erfindung wird vorzugsweise eine mehrfarbige Litze eingesetzt, für deren Isolierung Lacke mit typischerweise bis zu vier Farben verwendet werden. Die mit verschiedenen Farben isolierten Drähtchen sind getrennt ansteuerbar. Pro Litze können vorzugsweise 4 Signalleitungen vorhanden sein.

Diese Aufbauart ermöglicht es, bei maximalem Komfort für den Benutzer Elektroden bzw. sonstige Sensoren weitgehend frei anzubringen und die Position der Elektroden/Sensoren und der Elektronik weitgehend zu entkoppeln. Hierbei spielt die Konstruktion der Bändchen eine Rolle. Um schwache elektrische Signale ohne Störeinflüsse über eine weite Strecke zu übertragen, kann ein Koaxialkabel eingesetzt werden, was aber teuer ist. Eine andere Möglichkeit ist die Verwendung von Kabeln mit verdrillten Adernpaaren ("Twisted Pair"). Dies sind Kabeltypen, bei denen die Adern paarweise miteinander verdrillt sind. Adernpaare können mit unterschiedlich starker Verdrillung und unterschiedlichem Drehsinn in einem Kabel verseilt werden. Verdrillte Adernpaare bieten besseren Schutz gegenüber äußeren magnetischen Wechselfeldern und elektrostatischen Beeinflussungen als Adern, die nur parallel geführt sind. Durch das Verdrillen der Adernpaare heben sich Beeinflussungen durch äußere Felder größtenteils gegenseitig auf. Verdrillte Adernpaare werden mit symmetrischen Signalen beaufschlagt, um am fernen Ende einer (längeren) Kabelstrecke die Differenz zwischen den Signalen der beiden Adern bilden zu können und um damit das sendeseitige Signal bestmöglich am Empfangsort rekonstruieren zu können.

Die dehnbaren Bändchen ermöglichen die Nutzung eines verdrillten Adernpaares, in das ein Litzenbündel mit verschiedenen Farben eingebracht wurde. Das Litzenbündchen ist verdrillt und kann somit verschiedene Signale tragen. Vergleichbares gilt für Drähtchen, die spiralförmig um eine dehnbare Seele gewickelt werden. Auf diese Weise können eventuell eingestrahlte Störungen mit einem spezifischen Aufbau herausgefiltert werden.

Die Verwendung von Metall als Leiter hat außerdem zur Folge, dass niedrigohmig gearbeitet werden kann. Trotz der Verwendung von Metall sind die Leitungsbändchen oder Fäden aufgrund der Struktur der Bändchen und Fäden mit wellen- bzw. sinusartigem bzw. spiralförmigem Verlauf stark dehnbar, so dass sie für den vorgesehenen Zweck geeigneter sind als die aus dem Stand der Technik bekannten Konstrukte.

## Patentansprüche

1. Textilprodukt, das mindestens ein elektrisches Hautkontaktelement und eine Öffnung aufweist, wobei das Hautkontaktelement teilweise oder vollständig auf der Innenseite des Textilprodukts angeordnet ist und in einem die Öffnung umgebenden Bereich mit der Innenseite des Textilprodukts verklebt ist oder aufgedruckt ist, weiterhin umfassend wenigstens ein Ableitungselement in Form eines dehnbaren leitfähigen textilen Elements, wobei das elektrische Hautkontaktelement
• als dehnbare Elektrode ausgestaltet ist und einen leitfähige Partikel oder Graphen aufweisenden dehnbaren Kunststoff umfasst, und/oder
• als Sensor zum Messen von Feuchte oder PH-Wert, als elektrochemischer oder optischer Sensor ausgestaltet ist,
**dadurch gekennzeichnet, dass** das Ableitungselement ein dehnbares Leitungsbändchen ist und nicht auf der dem Körper zugewandten Seite des Textilprodukts angebracht ist und im Bereich der Öffnung mittels eines leitfähigen Silikons eine Verbindung mit der metallischen Seele des dehnbaren Leitungsbändchens hergestellt ist.

2. Textilprodukt, das mindestens ein elektrisches Hautkontaktelement aufweist, welches teilweise oder vollständig auf der Innenseite des Textilprodukts angeordnet ist oder das Gewebe des Textilprodukts durchsetzt, weiterhin umfassend wenigstens ein Ableitungselement in Form eines dehnbaren leitfähigen textilen Elements,
**dadurch gekennzeichnet, dass** das Ableitungselement auf der nicht dem Körper zugewandten Seite des Textilprodukts angebracht ist, eine Stelle im Textil mit einem leitfähigen Polymermaterial durchtränkt ist, so dass diese durchtränkte Stelle elektrisch leitfähig ist und einen Strom/eine Spannung von innen nach außen (oder umgekehrt) leiten kann und das Ableitungselement an der nicht dem Körper zugewandten Seite der Stelle mit dem leitfähigen Polymermaterial verklebt ist.

3. Textilprodukt nach einem der Ansprüche 1 bis 2, wobei die elastische Dehnbarkeit der Elektrode mindestens 10 %, vorzugsweise mindestens 20 %, stärker bevorzugt mindestens 30 % ist.

4. Textilprodukt nach einem der Ansprüche 1 bis 3, **dadurch gekennzeichnet, dass** das Hautkontaktelement als flächiger Druck-, Temperatur- oder Dehnungssensor ausgestaltet ist, der teilweise oder vollständig auf der Innenseite des Textilprodukts angeordnet ist, wobei der Sensor über ein leitfähiges, vorzugsweise dehnbares Material mit einem Ableitungselement verbunden ist, das auf der dem Körper des Trägers abgewandten Seite des Sensors angebracht ist.

5. Textilprodukt nach Anspruch 1 oder 2, **dadurch gekennzeichnet, dass** das Hautkontaktelement als Aktuator ausgestaltet ist.

6. Textilprodukt nach einem der Ansprüche 1 bis 5, wobei eine leitfähige Komponente des dehnbaren leitfähigen textilen Elements aus einem oder mehreren Metalldrähten besteht, die wellenförmig oder sinusförmig am Ableitungselement angebracht sind, oder bei dem eine oder mehrere dehnbare Seelen mit Metalldrähten umwunden sind.

7. Textilprodukt nach Anspruch 6, enthaltend mehrere einzelne oder paarweise verdrillte metallische Drähte als Leitungskomponente des Ableitungselements, die jeweils mit einer Isolierung versehen sind, wobei Drähte, die sich getrennt elektrisch ansteuern lassen, vorzugsweise eine unterschiedliche Farbe besitzen.

8. Textilprodukt nach einem der Ansprüche 1 - 7, worin das Ableitungselement auf der nicht dem Körper zugewandten Seite des Textilprodukts aufgeklebt, auflaminiert oder mit einem auflaminierten Band abgedeckt ist.

9. Textilprodukt nach einem der Ansprüche 1 - 7, worin das Ableitungselement in einer Flachnaht auf der nicht dem Körper zugewandten Seite des Textilprodukts angebracht oder, wobei das Ableitelement bevorzugt so dimensioniert ist, dass es für den Einsatz einer Flachnaht- Nähmaschine angepasst ist.

10. Verwendung eines Textilprodukts nach einem der Ansprüche 1 bis 9 zur Messung der Herzfrequenz, zum Erstellen eines Elektrokardiogramms, zum Aufbringen von Reiz- oder Stimulationsströmen oder zum Messen von Gehirnströmen.

11. Verfahren zum Herstellen eines Textilprodukts nach einem der vorhergehenden Ansprüche, welches die folgenden Schritte umfasst:
- Schritt (a), Erzeugen einer Öffnung oder eines leitfähigen Bereichs in einem Textilmaterial;
- Schritt (b), Anordnen und Verkleben oder Aufdrucken mindestens eines elektrischen Hautkontaktelements teilweise oder vollständig auf der Innenseite des Textilprodukts, wobei
∘ das elektrische Hautkontaktelement als dehnbare Elektrode ausgestaltet ist und einen leitfähige Partikel oder Graphen aufweisenden dehnbaren Kunststoff umfasst und/oder
∘ als Sensor zum Messen von Feuchte oder PH-Wert, als elektrochemischer oder optischer Sensor ausgestaltet ist;
- Schritt (c) Anbringen eines Ableitungselements in Form eines dehnbaren, als Leitungsbändchen ausgestalteten leitfähigen textilen Elements auf der nicht dem Körper zugewandten Seite des Textilprodukts; und
- Schritt (d) Herstellen eines Kontakts zwischen dem Ableitungselement und dem elektrischen Hautkontaktelement.

12. Verfahren nach Anspruch 11, wobei in Schritt (d) im Bereich der Öffnung mittels eines leitfähigen Silikons eine Verbindung mit der metallischen Seele des dehnbaren Leitungsbändchens hergestellt wird.

13. Verfahren nach Anspruch 11, wobei in Schritt (a) eine Öffnung in dem Textilmaterial erzeugt wird, auf einer Seite des Textilmaterials ein Klebemittel um die Öffnung herum aufgetragen wird, auf der anderen Seite der Öffnung ein die Öffnung abdeckender Träger bereitgestellt wird; das elektrische Hautkontaktelement auf das Klebemittel und den Träger aufgebracht wird; der Träger entfernt wird.

14. Verfahren nach Anspruch 11 oder 12, wobei das Hautkontaktelement und die zum Herstellen des Kontakts genutzte Verbindungsstelle mit nichtleitendem Material abgedeckt werden.

15. Verfahren nach Anspruch 11, wobei in Schritt (a) eine Öffnung in dem Textilmaterial erzeugt wird; und in Schritt (d) das Hautkontaktelement über ein leitfähiges Material mit metallischen Drähten eines dehnbaren Ableitungselements verbunden wird, bevor in Schritten (b) und (c) das mit dem Ableitungselement verbundene Hautkontaktelement oder ein Teil des Hautkontaktelements durch die Öffnung geschoben wird und dann mit Hilfe eines nichtleitenden Materials auf der Innenseite verklebt wird.

16. Verfahren nach einem der Ansprüche 11 - 15, wobei in Schritt (a) zum Herstellung eines leitfähigen Bereichs in dem Textilmaterial zumindest ein Teil eines dehnbaren Textilmaterials mit Elektrodenmaterial oder leitfähigem Material, das nach Härtung dehnbar ist und leitfähige Partikel enthält, versetzt, vorzugsweise durchtränkt wird, der leitfähige Bereich auf der nicht dem Körper zugewandten Seite des Textilmaterials mit der metallischen Seele des Leitungsbändchens verbunden wird und das Elektrodenmaterial gehärtet wird oder das leitfähige Material gehärtet und darauf auf der Innenseite des Textilmaterials ein Hautkontaktelement aufgebracht wird.

## Claims

1. A textile product comprising at least one electrical skin contact element and an opening, wherein the skin contact element is partially or completely arranged on the inside of the textile product and is adhered to or printed on the inside of the textile product in a region surrounding the opening, further comprising at least one conductive element in the form of an elastic conductive textile element, wherein the electric skin contact element
• is configured as an elastic electrode and comprises an elastic plastic comprising conductive particles and/or graphene, and/or
• is configured as a sensor for measuring the humidity or the pH value, as an electrochemical sensor or as an optical sensor,
**characterized in that** the conductive element is an elastic conductive tape and is not arranged on the side of the textile product facing the body, and that there is a connection with the metallic core of the elastic conductive tape by means of a conductive silicone in the region of the opening.

2. A textile product, comprising at least one electrical skin contact element which is partially or completely arranged on the inside of the textile product, or which penetrates the fabric of the textile product, further comprising at least one conductive element in the form of an elastic conductive textile element, **characterized in that** the conductive element is arranged on the side of the textile product not facing the body, a site in the textile is impregnated with a conductive polymer material so that this impregnated site is electrically conductive and can conduct a current/a voltage from the inside to the outside (or vice versa), and the conductive element is adhered to the conductive polymer material at the side of the site which is not facing the body.

3. The textile product according to claim 1 or 2, wherein the elastic stretch of the electrode is at least 10 %, preferably at least 20 %, further preferably at least 30 %.

4. The textile product according to any one of claims 1 to 3, **characterized in that** skin contact element is configured as a planar pressure sensor, temperature sensor or strain sensor which is partially or completely arranged on the inside of the textile product, the sensor being connected via a conductive, preferably elastic material to a conductive element which is mounted on the side of the sensor facing away from the body of the wearer.

5. The textile product according to claim 1 or 2, **characterized in that** the skin contact element is configured as an actuator.

6. The textile product according to any one of claims 1 to 5, wherein a conductive component of the elastic conductive textile element consists of one or more metal wires undulatingly or sinusoidally attached to the conductive element, or wherein one or more elastic cores are wrapped with metal wires.

7. The textile product according to claim 6, containing a plurality of single metallic wires or pairwise twisted metallic wires as a conductor component of the conductive element, each of which is provided with an insulation, wherein wires which can be electrically controlled separately preferably have a different colour.

8. The textile product according to any one of claims 1 - 7, wherein the conductive element is adhered to, laminated on or covered with a laminated tape on the side of the textile product not facing the body.

9. The textile product according to one of claims 1 - 7, wherein the conductive element is attached in a flat seam on the side of the textile product not facing the body, or wherein the conductive element is preferably dimensioned so as to be adapted for use with a flatlock sewing machine.

10. A use of a textile product according to any of claims 1 to 9 for measuring the heart rate, for generating an electrocardiogram, for applying stimulus currents or stimulation currents, or for measuring brain currents.

11. A process for the manufacture of a textile product according to any of the preceding claims, comprising the following steps:
step (a), generating an opening or a conductive region in a textile material;
step (b), arranging and adhering or printing at least one electrical skin contact element partially or completely on the inside of the textile product; wherein
• the electrical skin contact element is configured as an elastic electrode and comprises an elastic plastic comprising conductive particles and/or graphene, and/or
• is configured as a sensor for measuring the humidity or the pH value, as an electrochemical sensor or as an optical sensor,
step (c) attaching a conductive element in the form of an elastic conductive textile element formed as a conductive tape to the side of the textile product not facing the body; and
step (d) forming a contact between the conductive element and the electrical skin contact element.

12. The method according to claim 11, wherein in step (d) a connection with the metallic core of the elastic conductive tape is established in the region of the opening by means of a conductive silicone.

13. The method according to claim 11, wherein in step (a) an opening is formed in the textile material, an adhesive is applied around the opening on one side of the textile material, a carrier covering the opening is provided on the other side of the opening; the electrical skin contact element is mounted on the adhesive and the carrier; and the carrier is removed.

14. The method according to claim 11 or 12, wherein the skin contact element and the connection site used for creating the contact are covered with a non-conductive material.

15. The method according to claim 11, wherein in step (a) an opening is formed in the textile material; and in step (d) the skin contact element is connected via a conductive material to metallic wires of an elastic conductive element, before the skin contact element connected to the conductive element, or a portion of the skin contact element, is inserted through the opening in steps (b) and (c) and then adhered to the inside by means of a non-conductive material.

16. The method according to any one of claims 11 - 15, wherein in step (a), for forming a conductive region in the textile material, at least a portion of an elastic textile material is admixed with, preferably impregnated with, an electrode material or conductive material which is elastic after curing and contains conductive particles, the conductive region on the side of the textile material not facing the body is connected to the metallic core of the conductive tape, and the electrode material is cured or the conductive material is cured and a skin contact element is applied thereon on the inside of the textile material.

## Revendications

1. Produit textile qui présente au moins un élément de contact avec la peau électrique et une ouverture, dans lequel l'élément de contact avec la peau est disposé partiellement ou complètement sur le côté intérieur du produit textile et est collé ou imprimé sur le côté intérieur du produit textile dans une zone entourant l'ouverture, comprenant en outre au moins un élément de dérivation sous forme d'un élément textile conducteur extensible, dans lequel l'élément de contact avec la peau électrique
• est conçu sous forme d'électrode extensible et comprend une matière plastique extensible présentant des particules conductrices ou du graphène, et/ou
• est conçu sous forme de capteur pour la mesure de l'humidité ou du PH, sous forme de capteur électrochimique ou optique,
**caractérisé en ce que** l'élément de dérivation est un ruban conducteur extensible et n'est pas appliqué sur le côté du produit textile tourné vers le corps et, **en ce qu'**il y a une connexion avec l'âme métallique du ruban conducteur extensible au moyen d'un silicone conducteur dans la zone de l'ouverture.

2. Produit textile qui présente au moins un élément de contact avec la peau électrique qui est disposé partiellement ou complètement sur le côté intérieur du produit textile ou qui traverse le tissu du produit textile, comprenant en outre au moins un élément de dérivation sous forme d'un élément textile conducteur extensible,
**caractérisé en ce que** l'élément de dérivation est appliqué sur le côté du produit textile non tourné vers le corps, **en ce qu'**un emplacement dans le textile est imprégné d'un matériau polymère conducteur de sorte que ledit emplacement imprégné est électriquement conducteur et peut conduire un courant/une tension de l'intérieur vers l'extérieur (ou inversement), et l'élément de dérivation est collé au matériau polymère conducteur sur le côté de l'emplacement qui n'est pas tourné vers le corps.

3. Produit textile selon l'une des revendications 1 à 2, dans lequel l'extensibilité élastique de l'électrode est d'au moins 10 %, de préférence d'au moins 20 %, de manière particulièrement préférée d'au moins 30 %.

4. Produit textile selon l'une des revendications 1 à 3, **caractérisé en ce que** l'élément de contact avec la peau est conçu sous forme d'un capteur de pression planaire, d'un capteur de température ou d'un capteur de déformation, lequel est disposé partiellement ou complètement sur le côté intérieur du produit textile, dans lequel le capteur est connecté à un élément de dérivation par l'intermédiaire d'un matériau conducteur, de préférence extensible, lequel élément de dérivation est appliqué sur le côté du capteur opposé au corps du porteur.

5. Produit textile selon la revendication 1 ou 2, **caractérisé en ce que** l'élément de contact avec la peau est conçu sous forme d'un actionneur.

6. Produit textile selon l'une des revendications 1 à 5, dans lequel un composant conducteur de l'élément textile conducteur extensible est constitué d'un ou plusieurs fils métalliques ondulés ou sinusoïdaux disposés sur l'élément de dérivation, ou dans lequel une ou plusieurs âmes extensibles sont enlacées avec des fils métalliques.

7. Produit textile selon la revendication 6, contenant plusieurs fils métalliques individuels ou torsadés par paires comme composant conducteur de l'élément de dérivation, lesquels sont chacun pourvus d'une isolation, dans lequel les fils qui peuvent être commandés électriquement séparément, possèdent de préférence une couleur différente.

8. Produit textile selon l'une des revendications 1 à 7, dans lequel l'élément de dérivation est collé à, laminé ou recouvert d'une bande laminée sur le côté du produit textile non tourné vers le corps.

9. Produit textile selon l'une des revendications 1 à 7, dans lequel l'élément de dérivation est appliqué dans une couture plate sur le côté du produit textile non tourné vers le corps ou dans lequel, de préférence, l'élément de dérivation est dimensionné de manière à être adapté à l'utilisation d'une machine à coudre à couture plate.

10. Utilisation d'un produit textile selon l'une des revendications 1 à 9 pour la mesure de la fréquence cardiaque, pour la création d'un électrocardiogramme, pour l'application de courants d'excitation ou de stimulation ou pour la mesure de courants cérébraux.

11. Procédé de fabrication d'un produit textile selon l'une des revendications précédentes, comprenant les étapes suivantes :
- étape (a), formation d'une ouverture ou d'une zone conductrice dans un matériau textile ;
- étape (b), disposition et collage ou impression d'au moins un élément de contact avec la peau électrique partiellement ou complètement sur le côté intérieur du produit textile, dans lequel
∘ l'élément de contact avec la peau électrique est conçu sous forme d'électrode extensible et comprend une matière plastique extensible présentant des particules conductrices et/ou du graphène, et/ou
∘ est conçu sous forme de capteur pour la mesure de l'humidité ou du PH, sous forme de capteur électrochimique ou optique ;
- étape (c), application d'un élément de dérivation sous forme d'élément textile conducteur extensible, conçu sous forme de ruban conducteur, sur le côté du produit textile non tourné vers le corps ; et
- étape (d), établissement d'un contact entre l'élément de dérivation et l'élément de contact avec la peau électrique.

12. Procédé selon la revendication 11, dans lequel, à l'étape (d), une connexion avec l'âme métallique du ruban conducteur extensible est établie dans la zone de l'ouverture au moyen d'un silicone conducteur.

13. Procédé selon la revendication 11, dans lequel, à l'étape (a), une ouverture est formée dans le matériau textile, un adhésif est appliqué autour de l'ouverture sur un côté du matériau textile, un support recouvrant l'ouverture est appliqué sur l'autre côté de l'ouverture ; l'élément de contact avec la peau électrique est appliqué sur l'adhésif et le support ; le support est retiré.

14. Procédé selon la revendication 11 ou 12, dans lequel l'élément de contact avec la peau et l'emplacement de connexion utilisé pour l'établissement du contact sont recouverts d'un matériau non conducteur.

15. Procédé selon la revendication 11, dans lequel, à l'étape (a), une ouverture est formée dans le matériau textile ; et à l'étape (d), l'élément de contact avec la peau est connecté à des fils métalliques d'un élément de dérivation extensible par l'intermédiaire d'un matériau conducteur avant qu'aux étapes(b) et (c), l'élément de contact avec la peau ou une partie de l'élément de contact avec la peau connecté à l'élément de dérivation ne soit inséré dans l'ouverture puis collé à l'aide d'un matériau non conducteur sur le côté intérieur.

16. Procédé selon l'une des revendications 11 à 15, dans lequel, à l'étape (a), pour l'établissement d'une zone conductrice dans le matériau textile, au moins une partie d'un matériau textile extensible est mélangé, de préférence imprégné, avec un matériau d'électrode ou un matériau conducteur qui est extensible après durcissement et qui contient des particules conductrices, la zone conductrice est connectée à l'âme métallique du ruban conducteur sur le côté du matériau textile non tourné vers le corps, et le matériau d'électrode est durci ou le matériau conducteur est durci et un élément de contact avec la peau est appliqué par-dessus sur le côté intérieur du matériau textile.
